# EUROPÄISCHE PATENTSCHRIFT

(11) **EP 2 493 850 B1**
(45) Veröffentlichungstag und Bekanntmachung des Hinweises auf die Patenterteilung: **16.07.2014**
(21) Anmeldenummer: 10778596.6
(22) Anmeldetag: 27.10.2010
(51) Int. Cl.: C07C 263/04, C07C 29/12, C07C 29/128, C07C 31/20, C07C 265/12, C07C 265/14

(54) **VERFAHREN ZUR KOPPELPRODUKTION VON DI- UND/ODER POLYISOCYANATEN UND GLYKOLEN**
METHOD FOR THE COMBINED PRODUCTION OF DIISOCYANATES AND/OR POLYISOCYANATES AND GLYCOLS
PROCÉDÉ DE PRODUCTION COUPLÉE DE DI- ET/OU POLYISOCYANATES ET GLYCOLS

(30) Priorität: 27.10.2009 EP 09174169; 10.12.2009 EP 09178732
(43) Veröffentlichungstag der Anmeldung: 05.09.2012
(73) Patentinhaber: BASF SE, 67056 Ludwigshafen (DE)
(72) Erfinder: BOCK, Michael, 67152 Ruppertsberg (DE); BAUMANN, Robert, 68529 Mannheim (DE); FRANZKE, Axel, 68161 Mannheim (DE); STROEFER, Eckhard, 68163 Mannheim (DE); THEIS, Gerhard, 67133 Maxdorf (DE)
(86) Internationale Anmeldenummer: PCT/EP2010/066220
(87) Internationale Veröffentlichungsnummer: WO 2011/051314

(56) Entgegenhaltungen:
- WO-A1-2008/129030
- WO-A1-2008/138784
- WO-A1-2009/115538
- US-A- 6 080 897
- US-B2- 6 380 419

## Beschreibung

Die Erfindung betrifft ein Verfahren zur simultanen Produktion von Di- und/oder Polyisocyanaten und Glykolen in einem integrierten Verfahren.

Di- und Polyisocyanate sind wichtige Rohstoffe vor allem für die Polyurethanindustrie. Polyisocyanate im Sinne dieser Schrift haben eine Funktionalität von mehr als 2.

Der klassische Syntheseweg zur Herstellung von Di- und/oder Polyisocyanaten ist die Phosgenierung von Di- und/oder Polyaminen. Insbesondere aufgrund der mit dem Einsatz von Phosgen verbundenen Sicherheitsproblematik gewinnt der alternative Herstellungsweg über die thermische Spaltung von Di- und/oder Polycarbamaten, auch als Carbamidsäuredi- oder -polyester oder Di- oder Polyurethane bekannt, zunehmend an Bedeutung.

Di- und/oder Polycarbamate werden überwiegend durch Umsetzung der entsprechenden Dialkylcarbonate mit einem Di- und/oder Polyamin oder einer Mischung von Aminen hergestellt.
Die Umsetzung des Di- oder Polyamins mit Dialkylcarbonaten kann in Gegenwart von Alkoholaten als Base erfolgen (siehe z. B. WO 2009/115538).

Hierbei kann das Dialkylcarbonat durch Umesterung eines Alkylencarbonats mit einem Monohydroxyalkohol gewonnen werden. Bei dieser Umesterung fällt das entsprechende Alkylenglykol, im Folgenden kurz als Glykol bezeichnet, als Koppelprodukt an.

Darüber hinaus kann das Alkylencarbonat auch durch Hydrolyse in das entsprechende Alkylenglykol überführt werden. Beide Wertprodukte, Alkylenglykole sowie Di- und/oder Polyisocyanate, werden ausgehend von demselben Edukt, dem entsprechenden Alkylencarbonat, erhalten, das wiederum aus dem entsprechenden Alkylenoxid hergestellt wird.

Insbesondere an Standorten mit World-Scale-Alkylenoxidanlagen, in der Regel mit Steamcrackern, ist es daher vorteilhaft, Verbundanlagen zur Verfügung zu stellen, die Verfahrensschritte zur Synthese der beiden obigen Koppelprodukte integrieren.

WO 2008/129030 beschreibt ein Verfahren zur simultanen Herstellung von 1,2-Alkylendiolen und Dialkylcarbonaten, wobei ein 1,2-Alkylenoxid mit Kohlendioxid in Gegenwart eines Katalysators in einem Schlaufenreaktor zu einem Gas/Flüssigkeits-Reaktionsgemisch umgesetzt wird, das in einer zweiten Verfahrensstufe mit einem Alkanol zu einer Reaktionsmischung, enthaltend ein 1,2-Alkylendiol und ein Dialkylcarbonat, umgesetzt wird, woraus in Verfahrensstufe 3 das 1,2-Alkylendiol und das Dialkylcarbonat abgetrennt werden. Hierbei sollte das eingesetzte gasförmige Kohlendioxid möglichst effizient genutzt werden

Ein weiteres Verfahren zur simultanen Herstellung von Alkylenglykol und Dialkylcarbonat ist aus US 6,380,419 bekannt. Danach wird in einer ersten Verfahrensstufe Ethylenoxid mit Kohlendioxid zu einem Ethylencarbonat enthaltenden Gemisch umgesetzt, das in einer zweiten Verfahrensstufe destillativ in einen hauptsächlich Ethylencarbonat enthaltenden Strom 1 und eine Mischung aus Ethylencarbonat, Ethylenglykol und Wasser als Strom 2 aufgetrennt wird. Strom 1 wird in einer dritten Verfahrensstufe mit einer eine Hydroxylgruppe enthaltenden Verbindung umgeestert unter Erhalt des entsprechenden Dialkylcarbonates, das in einer vierten Verfahrensstufe destillativ gereinigt wird. Das dabei als Koppelprodukt gewonnene Ethylenglykol wird mit Strom 2 vereinigt, der in einer fünften Verfahrensstufe komplett hydrolysiert wird. Das daraus resultierende Ethylenglykol wird abschließend in einer sechsten Verfahrensstufe durch Destillation gereinigt. Nach diesem Verfahren wird somit das durch Umsetzung von Alkylenoxid mit Kohlendioxid erhaltene, Ethylencarbonat enthaltende Gemisch vor der Umesterung zum entsprechenden Dialkylcarbonat zunächst destillativ aufgereinigt.

Es war demgegenüber Aufgabe der Erfindung, ein technisch einfaches, kostengünstiges Verfahren zur Verfügung zu stellen, wonach ausgehend von Alkylenoxiden die Koppelprodukte Glykole und Di- und/oder Polyisocyanate in einfacher und kostengünstiger Weise gewonnen werden können.

Die Aufgabe wird gelöst durch ein Verfahren zur Koppelproduktion von Di- und/oder Polyisocyanaten und Glykolen, umfassend die Verfahrensstufen A, B, C und E zur Herstellung von Glykolen und die Verfahrensstufen A, C, D, E, F und G zur Herstellung von Di- und/oder Polyisocyanaten,
dadurch gekennzeichnet, dass
die stoffliche Kopplung über die Auftrennung des in Verfahrensstufe A erhaltenen Reaktionsgemisches in die Verfahrensstufen B und C erfolgt, wobei
- in der Verfahrensstufe A ein wässriges Alkylenoxid, das 30 bis 60 Gew.-% Wasser enthält, mit Kohlendioxid zu einem Alkylencarbonat enthaltenden Reaktionsgemisch umgesetzt wird,
- ein Teil des in Verfahrensstufe A erhaltenen, Alkylencarbonat enthaltenden Reaktionsgemisches in Verfahrensstufe B zu Glykol hydrolysiert wird,
- der übrige, Alkylencarbonat enthaltende Strom der Reaktionsmischung aus Verfahrensstufe A in Verfahrensstufe C entwässert wird,
- in Verfahrensstufe D Amin durch Hydrierung einer aromatischen Nitroverbindung oder eines Nitriles synthetisiert wird,
- in Verfahrensstufe E das entwässerte, Alkylencarbonat enthaltende Gemisch aus Verfahrensstufe C mit einem Monohydroxyalkohol zu dem entsprechenden Dialkylcarbonat umgeestert wird, wobei als Koppelprodukt Glykol erhalten wird,
- in Verfahrensstufe F das in Verfahrensstufe E erhaltene, Dialkylcarbonat enthaltende Reaktionsgemisch mit in Verfahrensstufe D erhaltenem Amin zu einem das entsprechende Mono-, Di und/oder Polycarbamat enthaltenden Gemisch umgesetzt wird, das
- in Verfahrensstufe G unter Erhalt der entsprechenden Di- und/oder Polyisocyanates gespalten wird.

Es wurde insbesondere gefunden, dass es möglich ist, das Alkylencarbonat ohne aufwändige Vorbehandlung, indem hieraus lediglich Wasser abgetrennt wird, der Umesterung zum entsprechenden Dialkylcarbonat zuzuführen. Es wurde gefunden, dass es ausreichend ist, lediglich das Reaktionswasser abzutrennen, eine Abreicherung der typischerweise in der Alkylencarbonatsynthese entstehenden Nebenprodukte, insbesondere der durch Hydrolyse des Alkylenoxids entstehenden Polyglykole sowie Abtrennung des gegebenenfalls vorhandenen homogenen Katalysators vor der Zuführung in die Umesterungsstufe nicht erforderlich ist. Die Rückstandsaufarbeitung kann deutlich vereinfacht werden, indem die Nebenprodukte und der Katalysator aus der Alkylencarbonatsynthese zusammen mit dem Rückstand aus der Umesterung (der Dialkylcarbonatsynthese) aufgearbeitet wird.

Insbesondere kann auch das in der Alkylencarbonatsynthese entstehende Alkylenglykol in der Verfahrensstufe der Umesterung (der Dialkylcarbonatsynthese) zusammen mit dem identischen Coprodukt der Umesterung abgetrennt werden.

Eine Wärmeintegration zwischen den Prozessstufen ist möglich und wirtschaftlich sinnvoll. In der vorliegenden Erfindung gibt es folgende großen Energiequellen: Wesentliche Energiequellen sind die Alkylencarbonatsynthese und die Herstellung von Aminen durch Hydrierung von Nitroaromaten oder Nitrilen. Wesentliche Energiesenken sind die Alkalialkoholatsynthese, die Umesterung von Alkylencarbonat und die Hydrolyse von Alkylencarbonat zu Glykol, wenn deutlich mehr Glykol als Isocyanat produziert wird. Die Reaktionswärmen der Alkylencarbonatsynthese und Aminbildung durch Hydrierung von Nitroaromaten oder Nitrilen können bevorzugt in der Urethanisierung für die Alkalialkoholatsynthese und/oder für die Umesterung in der Dialkylcarbonatsynthese verwendet werden. Beide Reaktionen sind sehr energieintensiv, da eine Gleichgewichtsverschiebung durch Destillation zugunsten der gewünschten Produkte stattfindet. Es ist auch möglich, z. B. 4 bar-Dampf in diesen beiden Stufen aus den exothermen Reaktionen zu erzeugen, der dann in der Urethanisierung (Alkalialkoholatsynthese) und/oder Umesterung zur Beheizung der Kolonnenverdampfer benutzt wird. Generell ist es möglich, die Alkylencarbonatsynthese (WO 2008/129030) und Hydrierungen zum Erhalt der Amine (z. B. WO 2008/138784 und DE 10349095) auf einem Temperaturniveau zu betreiben, so dass die Erzeugung von >=4 bar Dampf möglich ist.

In einer Hydrierung von Nitroaromaten rektifikativ gewonnenes Reaktionswasser kann für die Hydrolyse des Alkylencarbonats zum Glykol in der Simultanproduktion und/oder in der Protonierungsreaktion des Metallcarbamats anteilig verwertet werden.

Rohes, wässriges Ethylenoxid (30-60 Gew.-% Wasser) kann für die phosgenfreie Herstellung von Di- und/oder Polyisocyanaten über Ethylencarbonat als Ausgangsstoff für eine Dialkylcarbonatsynthese verwendet werden. Dadurch kann auf die aufwändige, energieintensive und mit umfangreichen Sicherheitsmaßnahmen verbundene Entwässerung des Ethylenoxids vor der Umsetzung mit Kohlendioxid verzichtet werden. Das erzeugte Ethylencarbonat wird erst nach der Synthese entwässert.

Als Kohlendioxidquelle für den Einsatz in Verfahrensstufe A kann insbesondere ein Abgas aus einer Ethylenoxidsynthese genutzt werden, in der Kohlendioxid durch Verbrennung von Ethylen mit Sauerstoff entsteht, oder auch das Abgas einer Synthesegasanlage.

In einer vorteilhaften Ausführungsform können die Verfahrensstufen A, B und C an einem Standort durchgeführt werden, der örtlich getrennt ist vom Standort der Verfahrensstufen D bis G. Eine derartige Verfahrensführung ist insbesondere attraktiv, wenn die Kapazität der Anlage zur Durchführung der Verfahrensstufen A, B und C wesentlich größer ist im Vergleich zur Kapazität der Anlagen für die Durchführung der weiteren Verfahrensstufen D bis G.

Die rohe Mischung der Alkylencarbonatsynthese wird nach der Entwässerung direkt der Carbonatsynthese zugeführt. Höhersiedende Nebenprodukte werden nicht abgetrennt.

Bevorzugt wird in Verfahrensstufe A als Alkylenoxid Ethylenoxid und/oder Propylenoxid verwendet.

In Verfahrensstufe A wird ein wässriges Alkylenoxid eingesetzt, das 30 bis 60 Gew.-% Wasser enthält.

Das in Verfahrensstufe D erhaltene Amin, das in Verfahrensstufe F mit dem in Verfahrensstufe E erhaltenen, Dialkylcarbonat enthaltenden Reaktionsgemisch zu einem das entsprechende Mono-, Di- und/oder Polycarbamat enthaltenden Gemisch umgesetzt wird, ist bevorzugt eine Einzelsubstanz oder eine Mischung von Substanzen oder ihrer Isomeren, ausgewählt aus der nachfolgenden Aufzählung: TDA (Toluylendiamin), MDA (Diaminodiphenylmethan), pMDA (Polyphenylenpolymethylenpolyamin), Anilin, HDA (Hexamethylendiamin), IPDA (Isophorondiamin), TMXDA (Tetramethylenxylylendiamin), NDA (Naphthylendiamin), H6TDA (Hexahydrotoluylendiamin), H12MDA (Diaminodicyclohexylmethan) und Diaminobenzol.

Der in Verfahrensstufe E eingesetzte Monohydroxyalkohol ist bevorzugt ein aliphatischer Alkohol, der 2 bis 10 Kohlenstoffatome und gegebenenfalls Sauerstoff- und/oder Stickstoffatome enthält.

Bevorzugt ist der aliphatische, 2 bis 10 Kohlenstoffatome enthaltende Monohydroxyalkohol verzweigt, insbesondere an dem Kohlenstoffatom, das unmittelbar dem Kohlenstoffatom benachbart ist, das die Hydroxylgruppe trägt.

In einer bevorzugten Ausführungsform der Erfindung wird aus dem in Verfahrensstufe F (Urethansynthese) erhaltenen Reaktionsgemisch das Carbamat abgetrennt, und dieses wird mit Formaldehyd oder einem Formaldehydderivat zu einem Polymere des Carbamates enthaltenden Gemisch kondensiert, woraus die Polycarbamate abgetrennt und der Verfahrensstufe G (Spaltung) zugeführt werden.

Vorteilhaft wird die Verfahrensstufe F in Gegenwart eines Katalysators durchgeführt.

Weiter bevorzugt wird die Verfahrensstufe F in Gegenwart eines inerten Lösungsmittels durchgeführt.

Die thermische Kopplung der Verfahrensstufen kann insbesondere durchgeführt werden, indem Reaktionswärme aus den Verfahrensstufen A und D in einer oder mehreren der Verfahrensstufen B, C, E und F genutzt wird.

Nachfolgend werden bevorzugte Varianten der energetischen Kopplung beschrieben.

### Wesentliche Energiequellen sind

1. die Alkylencarbonatsynthese (stark exotherm) (Verfahrensstufe A),
2. die Herstellung von Aminen durch Hydrierung von Nitroaromaten (sehr stark exotherm) (Verfahrensstufe D),
3. die Abwärme des Carbamatspaltgases G bis zum Taupunkt (Verfahrensstufe G) sowie
4. die Kondensationswärme der Alkylencarbonatentwässerung (Verfahrensstufe C), wenn Alkylencarbonat anteilig zur Herstellung von reinem Alkylencarbonat im Abtriebsteil der Rektifikation gasförmig entnommen wird.

### Wesentliche Energiesenken bei niedriger Temperatur sind

1. die Alkalialkoholatsynthese (Gleichgewichtsverschiebung, sehr hoher Dampfbedarf) (Verfahrensstufe F, Bestandteil der Urethanisierung),
2. die Umesterung von Alkylencarbonat zu Dialkylcarbonat (Gleichgewichtsverschiebung, sehr hoher Dampfbedarf) (Verfahrensstufe E),
3. die Hydrolyse von Alkylencarbonat zu Glykol (endotherm) (Verfahrensstufe B),
4. die Trennung von Wasser und Glykol nach der Hydrolyse, wenn deutlich mehr Glykol als Isocyanat produziert wird (Verfahrensstufe B) sowie
5. die Beheizung des Sumpfes bei der Entwässerung des Alkylencarbonats (Verfahrensstufe C).

Die oben genannten Energiesenken arbeiten typischerweise mindestens 10 °C unter den genannten Energiequellen. Somit kann die Energie übertragen werden. Es gibt also neben der stofflichen auch eine vorteilhafte energetische Kopplung zwischen den Prozessstufen.

Vorteilhaft kann mindestens ein Teil der Reaktionswärme der Alkylencarbonatsynthese (Verfahrensstufe A) zur Alkalialkoholatsynthese in der Urethanisierung (Verfahrensstufe F) genutzt werden.

Vorteilhaft kann mindestens ein Teil der Reaktionswärme der Alkylencarbonatsynthese (Verfahrensstufe A) zur Umesterung des Alkylencarbonats (Verfahrensstufe E) verwendet werden.

Vorteilhaft kann mindestens ein Teil der Reaktionswärme der Aminbildung durch Hydrierung (Verfahrensstufe D) zur Alkalialkoholatsynthese in der Urethanisierung (Verfahrensstufe F) genutzt werden.

Vorteilhaft kann mindestens ein Teil der Reaktionswärme der Aminbildung durch Hydrierung (Verfahrensstufe D) zur Umesterung des Alkylencarbonats (Verfahrensstufe E) verwendet werden.

Vorteilhaft kann mindestens ein Teil der Kondensationswärmen einer Alkylencarbonatentwässerung (Verfahrensstufe C) bei der Alkalialkoholatsynthese in der Urethanisierung (Verfahrensstufe F) genutzt werden.

Insbesondere kann mindestens ein Teil der Reaktionswärme der Alkylencarbonatsynthese (Verfahrensstufe A) in der Carbamatspaltung (Verfahrensstufe G) verwendet werden.

Vorteilhaft kann mindestens ein Teil der Reaktionswärme der Aminbildung durch Hydrierung (Verfahrensstufe D) zur Beheizung der Alkylencarbonathydrolyse (Verfahrensstufe B) und/oder der Entwässerung des Alkylencarbonats (Verfahrensstufe C) verwendet werden.

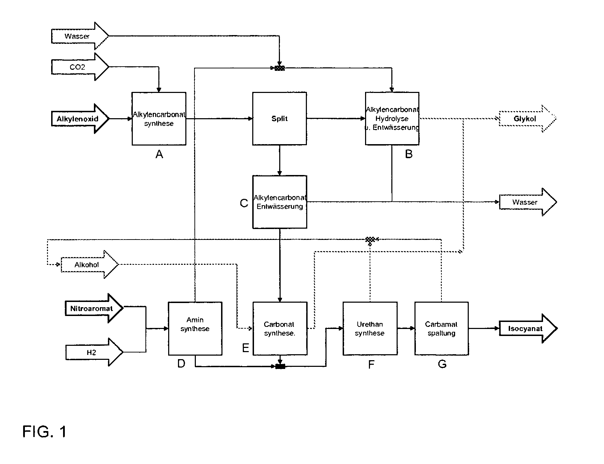

Die Erfindung wird im Folgenden anhand der Figur 1 sowie eines Ausführungsbeispiels für den exemplarischen Fall der Koppelproduktion von Ethandiol ((CH₂OH)₂) und Toluylendiisocyanat (TDI) aus Ethylenoxid (EO) und Dinitrotoluol (DNT) näher erläutert.

### Ausführungsbeispiel:

### Stufen A bis C, E

53200 kg/h wässriges Ethylenoxid (44,8 Gew.-% Wasser) werden bei 110 °C mit 34186 kg/h Kohlendioxid in Gegenwart von einem stark basischen Anionenaustauscher zu Ethylencarbonat und Ethandiol umgesetzt (Verfahrensstufe A). 50% des Rohprodukts werden in Verfahrensstufe C überführt und 50% des Rohprodukts in Verfahrensstufe B. Als Nebenreaktion entstehen in Verfahrensstufe A Ethandiol und höhere Glykole.

In Verfahrensstufe B entstehen nach Hydrolyse und Entwässerung 20298 kg/h Ethandiol.

Der aus Verfahrensstufe A abgetrennte nicht hydrolysierte Reaktionsaustrag (42099 kg/h, 66,7 Gew.-% Ethylencarbonat) wird entwässert, ohne dass die polymeren Nebenprodukte der Synthese abgetrennt werden. Das entwässerte Ethylencarbonat wird dann bei 160 °C in Gegenwart von 1 mol% Natriumisobutylat in einem Rohrreaktor mit 25942 kg/h Isobutanol zu 54014 kg/h Diisobutylcarbonat und 19250 kg/h Ethandiol umgesetzt (Verfahrensstufe E). Nicht umgesetzte Komponenten (wie Ethylencarbonat und Isobutanol) werden über eine Vakuumdestillation in den Gleichgewichtsreaktor zurückgeführt. Die Produkte (Diisobutylcarbonat und Ethandiol) werden ebenfalls im Vakuum destillativ voneinander getrennt.

Die gemeinsamen Rückstände von Verfahrensstufe A und Verfahrensstufe E werden gemeinsam in Verfahrensstufe E ausgeschleust.

Das in Verfahrensstufe A durch die partielle Hydrolyse von Ethylenoxid in geringen Mengen entstandene Ethandiol wird ebenfalls hier (Verfahrensstufe E) mit dem in der Umesterung von Ethylencarbonat zu Diisobutylcarbonat zusätzlich als Koppelprodukt entstandenen Ethandiol ausgeschleust und mit dem Ethandiol aus Verfahrensstufe B vereinigt.

### Stufe D

29870 kg/h technisches Dinitrotoluol (DNT) wird bei 180 °C unter Nutzung von 2180 kg/h Wasserstoff zu 18936 kg/h technischem Toluylendiamin (TDA) umgesetzt. 385 kg/h schwersiedende Nebenprodukte werden vom TDA nach zuvor erfolgter Entwässerung in einer Trennwandkolonne abgetrennt.

### Stufe F

Die Verfahrensstufe F besteht aus der Urethanisierung und der Alkalialkoholatherstellung. Dazu werden 24878 kg/h 50 w% Natronlauge mit 120639 kg/h Isobutanol bei 90 °C in einer Reaktionskolonne unter Wasserausschleusung zu insgesamt 29860 kg/h Natriumisobutylat gelöst in Isobutanol umgesetzt (20w% Lösung). Das in Stufe E gewonnene Diisobutylcarbonat (54014 kg/h) wird mit diesen 29860 kg/h (ber. 100%) Natriumisobutylat in Isobutanol (20 Gew.-%) und 18936 kg/h TDA-Schmelze aus Stufe D bei 120 °C zur Reaktion gebracht (Verfahrensstufe F, Schritt 1) und in einer nachfolgenden Hydrolyse mit Wasser bei 50 °C zu 49975 kg/h Toluylen-bis(O-isobutylcarbamat) umgesetzt (Verfahrensstufe F, Schritt 2). Die Reaktionsmischung aus Schritt 2 der Stufe F wird nach einer extraktiven Abtrennung der Natronlauge anschließend durch Destillation der organischen Phase in Dicarbamat, Diisobutylcarbonat und Isobutanol aufgetrennt. Natronlauge und Isobutanol werden entsprechend in die Isobutylatsynthese und die Umesterung zurückgeführt.

### Stufe G

Das Carbamat wird als 130 °C heiße Schmelze in eine Wirbelschicht dosiert und bei 400 °C in Toluylendiisocyanat (TDI) und Alkohol gespalten, wobei Stickstoff als Wirbelgas verwendet wird (Verfahrensstufe G). Die gasförmige Produktmischung wird in einem Flüssigquench unter den Taupunkt von TDI abgekühlt und anschließend durch Destillation im Vakuum in TDI, Isobutanol und Quenchmedium aufgetrennt. Isobutanol wird rückgeführt.

Somit entstehen insgesamt 25143 kg/h TDI und 39548 kg/h Ethandiol als Wertprodukte.

Nachfolgend erfolgt die Beschreibung der energetischen Kopplung des Verfahrens. *Wesentliche Energiequellen bei höherer Temperatur sind*
1. die Ethylencarbonatsynthese (stark exotherm, 110 °C) (Stufe A)
2. die Herstellung von TDA durch Hydrierung von DNT (sehr stark exotherm, 180 °C) (Stufe D)
3. die Abwärme des Carbamatspaltgases im Quench bis zum Taupunkt (Stufe G) und
4. die Kondensationswärme der Ethylencarbonatentwässerung (Stufe C) bei Abzug von gasförmigen Ethylencarbonat als Nebenprodukt bei einem Betriebsdruck von 100mbar abs oder weniger (bis 30 mbar)

### Wesentliche Energiesenken bei niedrigerer Temperatur sind

a. die Natriumisobutylatsynthese (sehr hoher Dampfbedarf, 90 °C) (Stufe F),
b. die Umesterung von Ethylencarbonat zu Diisobutylcarbonat (sehr hoher Dampfbedarf,) (Stufe E). Ethylencarbonat wird auf Reaktionstemperatur von 160°C vorgewärmt (Möglich: 100 bis 160°C). Betrieb von zwei Rektifikationskolonnen zur Reindestillation von Diisobutylcarbonat und Ethandiol bei jeweils weniger als 300 mbar abs.
c. die Hydrolyse von Ethylencarbonat zu Ethandiol (endotherm, unter 160 °C, s. US 6,080,897, Beispiel 3, Spalte 10) (Stufe B),
d. die Trennung von Wasser und Ethandiol nach der Hydrolyse, s. US 6,080,897, Beispiel 3, Spalte 10 (Stufe B) sowie
e. die Sumpfbeheizung bei der Entwässerung des Ethylencarbonats, da diese bei einem Druck von 100 mbar abs oder weniger (bis 30 mbar) betrieben wird. (Stufe C).

Die oben genannten Energiesenken arbeiten mindestens 10 °C unter den genannten Energiequellen. Somit kann die Energie übertragen werden. Es gibt also neben der stofflichen auch eine vorteilhafte energetische Kopplung zwischen den einzelnen Verfahrensstufen untereinander.

Vorteilhaft kann somit folgender Wärmeverbund realisiert werden:
Quelle 2 (Dampf aus der DNT-Hydrierung) wird ganz oder teilweise zur Beheizung der Senken a, b, c, d und/oder e verwendet.
Quelle 1 (Abwärme aus der Ethylencarbonatsynthese) wird t ganz oder teilweise zur Beheizung der Senke a verwendet.
Quelle 3 (Abwärme aus Quench / Abkühlen des Carbamatspaltgases) wird ganz oder teilweise zur Beheizung der Senke b verwendet.
Quelle 4 (Abwärme aus Entwässerung bei gasförmigem Seitenabzug) wird ganz oder teilweise zur Beheizung der Senke a verwendet.

Die Figur 1 zeigt die schematische Darstellung einer bevorzugten Anlage zur Durchführung des erfindungsgemäßen Verfahrens:
In Verfahrensstufe A wird ein wässriges Alkylenoxid, beispielhaft wässriges Ethylenoxid (EO) mit Kohlendioxid zu einem Alkylencarbonat enthaltenden Reaktionsgemisch umgesetzt.

Das in Verfahrensstufe A erhaltene, das Alkylencarbonat enthaltende Reaktionsgemisch wird aufgeteilt ("Split") in einen Strom, der in Verfahrensstufe B zu Glykol, beispielhaft Ethandiol ((CH₂OH)₂), hydrolysiert wird, und in einen übrigen Strom, der in Verfahrensstufe C entwässert wird.

In Verfahrensstufe D wird durch Hydrierung einer aromatischen Nitroverbindung, beispielhaft Dinitrotoluol (DNT), ein Amin, beispielhaft Toluylendiamin (TDA), synthetisiert.

In Verfahrensstufe E wird das entwässerte, das Alkylencarbonat enthaltende Gemisch aus Verfahrensstufe C mit einem Monohydroxyalkohol, beispielhaft Isobutanol, zu dem entsprechenden Dialkylcarbonat umgeestert, wobei als Koppelprodukt Glykol, beispielhaft Ethandiol ((CH₂OH)₂), erhalten wird.

In Verfahrensstufe F wird das in Verfahrensstufe E erhaltene, das Dialkylcarbonat enthaltende Reaktionsgemisch mit dem in Verfahrensstufe D erhaltenen Amin zu einem das entsprechende Carbamat enthaltenden Gemisch umgesetzt, das in Verfahrensstufe G unter Erhalt des entsprechenden Isocyanats, beispielhaft Toluylendiisocyanat (TDI), gespalten wird.

## Patentansprüche

1. Verfahren zur Koppelproduktion von Di- und/oder Polyisocyanaten und Glykolen, umfassend die Verfahrensstufen A, B, C und E zur Herstellung von Glykolen und die Verfahrensstufen A, C, D, E, F und G zur Herstellung von Di- und/oder Polyisocyanaten,
**dadurch gekennzeichnet, dass**
die stoffliche Kopplung über die Auftrennung des in Verfahrensstufe A erhaltenen Reaktionsgemisches in die Verfahrensstufen B und C erfolgt, wobei
- in der Verfahrensstufe A ein wässriges Alkylenoxid, das 30 bis 60 Gew.-% Wasser enthält, mit Kohlendioxid zu einem Alkylencarbonat enthaltenden Reaktionsgemisch umgesetzt wird,
- ein Teil des in Verfahrensstufe A erhaltenen, Alkylencarbonat enthaltenden Reaktionsgemisches in Verfahrensstufe B zu Glykol hydrolysiert wird,
- der übrige, Alkylencarbonat enthaltende Strom der Reaktionsmischung aus Verfahrensstufe A in Verfahrensstufe C entwässert wird,
- in Verfahrensstufe D Amin durch Hydrierung einer aromatischen Nitroverbindung oder eines Nitriles synthetisiert wird,
- in Verfahrensstufe E das entwässerte, Alkylencarbonat enthaltende Gemisch aus Verfahrensstufe C mit einem Monohydroxyalkohol zu dem entsprechenden Dialkylcarbonat umgeestert wird, wobei als Koppelprodukt Glykol erhalten wird, und
- in Verfahrensstufe F das in Verfahrensstufe E erhaltene, Dialkylcarbonat enthaltende Reaktionsgemisch mit in Verfahrensstufe D erhaltenem Amin zu einem das entsprechende Mono-, Di- und/oder Polycarbamat enthaltenden Gemisch umgesetzt wird, das
- in Verfahrensstufe G unter Erhalt des entsprechenden Di- und/oder Polyisocyanates gespalten wird.

2. Verfahren nach Anspruch 1, **dadurch gekennzeichnet, dass** das in Verfahrensstufe A eingesetzte Alkylenoxid Ethylenoxid und/oder Propylenoxid ist.

3. Verfahren nach einem der Ansprüche 1 oder 2, **dadurch gekennzeichnet, dass** das in Verfahrensstufe D erhaltene Amin eine Einzelsubstanz oder eine Mischung von Substanzen oder ihrer isomeren, ausgewählt aus der nachfolgenden Aufzählung ist: TDA (Toluylendiamin), MDA (Diaminodiphenylmethan), pMDA (Polyphenylenpolymethylenpolyamin), HDA (Hexamethlyendiamin), IPDA (Isophorondiamin). TMXDA (Tetramethylenxylyfendianlin), NDA (Naphthylendiamin), H6TDA (Hexahydrotoluylendlamin), H12MDA (Diaminodicyclohexylmethan) und Diaminobenzol.

4. Verfahren nach einem der Ansprüche 1 bis 4, **dadurch gekennzeichnet, dass** der in Verfahrensstufe E eingesetzte Monohydrokyalkohol ein aliphatischer Alkohol ist, der 2 bis 10 Kohlenstoffatome und gegebenenfalls Sauerstoff- und/oder Stickstoffatome enthält.

5. Verfahren nach Anspruch 4, **dadurch gekennzeichnet, dass** der aliphatische, 2 bis 10 Kohlenstoffatome enthaltende Monohydroxyalkohol verzweigt ist, insbesondere an dem Kohlenstoffatom, das unmittelbar dem Kohlenstoffatom benachbart ist, das die Hydroxylgruppe trägt.

6. Verfahren nach einem der Ansprüche 1, 2, 4 oder 5, **dadurch gekennzeichnet, dass** aus dem in Verfahrensstufe F (Urethansynthese) erhaltenen Reaktionsgemisch das Carbamat abgetrennt, mit Formaldehyd oder einem Formaldehydderivat zu einem Polymere des Carbamates enthaltenden Gemisch kondensiert wird, woraus die Polycarbamate abgetrennt und der Verfahrensstufe G (Spaltung) zugeführt werden.

7. Verfahren nach Anspruch 6, **dadurch gekennzeichnet, dass** in Verfahrensstufe D enthaltenes Amin Anilin ist.

8. Verfahren nach einem der Ansprüche 1 bis 7, **dadurch gekennzeichnet, dass** die Verfahrensstufe F in Gegenwart eines Katalysators durchgeführt wird.

9. Verfahren nach einem der Ansprüche 1 bis 8, **dadurch gekennzeichnet, dass** die Verfahrensstufe F in Gegenwart eines inerten Lösungsmittels durchgeführt wird.

10. Verfahren nach einem der Ansprüche 1 bis 9, **dadurch gekennzeichnet, dass** die Reaktionswärme aus den Verfahrensstufen A und D in einer oder mehreren der Verfahrensstufen B, C, E und F genutzt wird.

## Claims

1. A process for coproducing di- and/or polyisocyanates and glycols, comprising process stages A, B, C and E for preparing glycols and process stages A, C, D, E, F and G for preparing di- and/or polyisocyanates,
which comprises
accomplishing the material coupling via the separation of the reaction mixture obtained in process stage A into process stages B and C, by
- in process stage A, reacting an aqueous alkylene oxide comprising 30 to 60% by weight of water with carbon dioxide to give a reaction mixture comprising alkylene carbonate,
- hydrolyzing a portion of the alkylene carbonate-comprising reaction mixture obtained in process stage A to glycol in process stage B,
- dewatering the remaining alkylene carbonate-comprising stream of the reaction mixture from process stage A in process stage C,
- in process stage D, synthesizing amine by hydrogenating an aromatic nitro compound or a nitrile,
- in process stage E, transesterifying the dewatered alkylene carbonate-comprising mixture from process stage C with a monohydroxy alcohol to give the corresponding dialkyl carbonate, obtaining glycol as a coproduct, and
- in process stage F, reacting the dialkyl carbonate-comprising reaction mixture obtained in process stage E with the amine obtained in process stage D to a mixture comprising the corresponding mono-, di- and/or polycarbamate, which
- in process stage G is cleaved to obtain the corresponding di- and/or polyisocyanate.

2. The process according to claim 1, wherein the alkylene oxide used in process stage A is ethylene oxide and/or propylene oxide.

3. The process according to either of claims 1 and 2, wherein the amine obtained in process stage D is an individual substance or a mixture of substances or isomers thereof, selected from the following list: TDA (tolylenediamine), MDA (diaminodiphenylmethane), pMDA (polyphenylenepolymethylenepolyamine), HDA (hexamethylenediamine), IPDA (isophoronediamine), TMXDA (tetramethylenexylylenediamine), NDA (naphthylenediamine), H6TDA (hexahydrotolylenediamine), H12MDA (diaminodicyclohexylmethane) and diaminobenzene.

4. The process according to any of claims 1 to 4, wherein the monohydroxy alcohol used in process stage E is an aliphatic alcohol which comprises 2 to 10 carbon atoms and optionally oxygen and/or nitrogen atoms.

5. The process according to claim 4, wherein the aliphatic monohydroxy alcohol comprising 2 to 10 carbon atoms is branched, especially at the carbon atom directly adjacent to the carbon atom which bears the hydroxyl group.

6. The process according to any of claims 1, 2, 4 and 5, wherein the carbamate is removed from the reaction mixture obtained in process stage F (urethane synthesis) and condensed with formaldehyde or a formaldehyde derivative to give a mixture comprising polymers of the carbamate, from which the polycarbamates are removed and sent to process stage G (cleavage).

7. The process according to claim 6, wherein amine present in process stage D is aniline.

8. The process according to any of claims 1 to 7, wherein process stage F is performed in the presence of a catalyst.

9. The process according to any of claims 1 to 8, wherein process stage F is performed in the presence of an inert solvent.

10. The process according to any of claims 1 to 9, wherein the heat of reaction from process stages A and D is utilized in one or more of process stages B, C, E and F.

## Revendications

1. Procédé de production couplée de di- et/ou polyisocyanates et de glycols, comprenant les étapes de procédé A, B, C et E pour la fabrication de glycols et les étapes de procédé A, C, D, E, F et G pour la fabrication de di- et/ou de polyisocyanates, **caractérisé en ce que**
le couplage matériel a lieu par la séparation du mélange réactionnel obtenu à l'étape de procédé A lors des étapes de procédé B et C,
- lors de l'étape de procédé A, un oxyde d'alkylène aqueux, qui contient 30 à 60 % en poids d'eau, étant mis en réaction avec du dioxyde de carbone pour former un mélange réactionnel contenant un carbonate d'alkylène,
- une partie du mélange réactionnel contenant un carbonate d'alkylène obtenu à l'étape de procédé A étant hydrolysée en glycol à l'étape de procédé B,
- le courant contenant un carbonate d'alkylène restant du mélange réactionnel de l'étape A étant déshydraté à l'étape de procédé C,
- lors de l'étape de procédé D, une amine étant synthétisée par hydrogénation d'un composé nitro aromatique ou d'un nitrile,
- lors de l'étape de procédé E, le mélange contenant un carbonate d'alkylène déshydraté issu de l'étape de procédé C étant transestérifié avec un monohydroxyalcool pour former le carbonate de dialkyle correspondant, du glycol étant obtenu en tant que sous-produit, et
- lors de l'étape de procédé F, le mélange réactionnel contenant un carbonate de dialkyle obtenu à l'étape de procédé E étant mis en réaction avec l'amine obtenue à l'étape de procédé D pour former un mélange contenant le mono-, di- et/ou polycarbamate correspondant, qui
- lors de l'étape de procédé G, est clivé pour obtenir le di- et/ou polyisocyanate correspondant.

2. Procédé selon la revendication 1, **caractérisé en ce que** l'oxyde d'alkylène utilisé à l'étape de procédé A est l'oxyde d'éthylène et/ou l'oxyde de propylène.

3. Procédé selon l'une quelconque des revendications 1 ou 2, **caractérisé en ce que** l'amine obtenue à l'étape de procédé D est une substance individuelle ou un mélange de substances ou leurs isomères, choisis dans la liste suivante : la TDA (toluylène diamine), le MDA (diaminodiphénylméthane), la pMDA (polyphénylène polyméthylène polyamine), l'HDA (hexaméthylène diamine), l'IPDA (isophorone diamine), la TMXDA (tétraméthylène xylylène diamine), la NDA (naphtylène diamine), l'H6TDA (hexahydrotoluylène diamine), l'H12MDA (diaminodicyclohexylméthane) et le diaminobenzène.

4. Procédé selon l'une quelconque des revendications 1 à 4, **caractérisé en ce que** le monohydroxyalcool utilisé à l'étape de procédé E est un alcool aliphatique qui contient 2 à 10 atomes de carbone et éventuellement des atomes d'oxygène et/ou d'azote.

5. Procédé selon la revendication 4, **caractérisé en ce que** le monohydroxyalcool aliphatique contenant 2 à 10 atomes de carbone est ramifié, notamment sur l'atome de carbone directement voisin de l'atome de carbone qui porte le groupe hydroxyle.

6. Procédé selon l'une quelconque des revendications 1, 2, 4 ou 5, **caractérisé en ce que** le carbamate est séparé à partir du mélange réactionnel obtenu à l'étape de procédé F (synthèse d'uréthane), condensé avec du formaldéhyde ou un dérivé de formaldéhyde en un mélange contenant des polymères du carbamate, les polycarbamates sont séparés de celui-ci et introduits dans l'étape de procédé G (clivage).

7. Procédé selon la revendication 6, **caractérisé en ce que** l'amine contenue à l'étape de procédé D est l'aniline.

8. Procédé selon l'une quelconque des revendications 1 à 7, **caractérisé en ce que** l'étape de procédé F est réalisée en présence d'un catalyseur.

9. Procédé selon l'une quelconque des revendications 1 à 8, **caractérisé en ce que** l'étape de procédé F est réalisée en présence d'un solvant inerte.

10. Procédé selon l'une quelconque des revendications 1 à 9, **caractérisé en ce que** la chaleur de réaction des étapes de procédé A et D est utilisée dans une ou plusieurs des étapes de procédé B, C, E et F.
